# EUROPEAN PATENT SPECIFICATION

(11) **EP 1 893 722 B1**
(45) Date of publication and mention of the grant of the patent: **10.05.2017**
(21) Application number: 05780594.7
(22) Date of filing: 17.08.2005
(51) Int. Cl.: C10G 7/08, C10G 21/27, C10G 45/58, C10G 45/64, C10G 47/00, C10G 61/08

(54) **PROCESS FOR INCREASING PRODUCTION OF BENZENE FROM HYDROCARBON MIXTURE**
VERFAHREN ZUR ERHÖHUNG DER PRODUKTION VON BENZOL AUS EINEM KOHLENWASSERSTOFFGEMISCH
PROCÉDÉ DESTINÉ À AUGMENTER LA PRODUCTION DE BENZÈNE À PARTIR D'UN MÉLANGE D'HYDROCARBURES

(30) Priority: 21.06.2005 KR 20050053619
(43) Date of publication of application: 05.03.2008
(73) Proprietor: SK Innovation Co., Ltd., Seoul 110-110 (KR)
(72) Inventor: OH, Seung Hoon, Daejeon 305-712 (KR); CHANG, Byoung Mu, Daejeon 305-728 (KR); CHOI, Sun, Daejeon 305-761 (KR); KANG, Sin Choel, Yangcheon-gu, Seoul 158-776 (KR); LEE, Jong Hyung, Daejeon 302-120 (KR); SUNG, Kyoung Hak, Daejeon 305-712 (KR); LIM, Byeung Soo, Daejeon 300-768 (KR); KIM, Yong Seung, Daejeon 305-810 (KR)
(74) Representative: Perani, Aurelio
(86) International application number: PCT/KR2005/002708
(87) International publication number: WO 2006/137616

(56) References cited:
- US-A- 3 702 293
- US-A- 3 729 409
- US-A- 4 058 454
- US-A- 4 167 533
- US-A- 5 278 344
- US-A1- 2002 092 797

## Description

### Technical Field

The present invention relates to a process for increasing the production of benzene from a hydrocarbon mixture. More particularly, the present invention pertains to a process for increasing the production of benzene by integrating a process for producing an aromatic hydrocarbon mixture and liquefied petroleum gas (LPG) from a hydrocarbon mixture with a solvent extraction process for separating and recovering polar hydrocarbons from the hydrocarbon mixture.

### Background Art

Generally, aromatic hydrocarbons are obtained by separating a feedstock fraction, which is rich in aromatic compounds, such as reformates produced through a catalytic reforming process and pyrolysis gasolines produced through a naphtha cracking process, from non-aromatic hydrocarbons using a solvent extraction process. The aromatic hydrocarbon mixture thus obtained is separated into benzene, toluene, xylene, and C9+ aromatic compounds using a difference in boiling point to use them as basic petrochemical materials, and the non-aromatic hydrocarbons are used as a feedstock or a fuel for the naphtha cracking process.

With respect to this, U.S. Patent No. 4,058,454 discloses a solvent extraction process for separating and recovering polar hydrocarbons from a hydrocarbon mixture containing the polar hydrocarbons and nonpolar hydrocarbons. Most solvent extraction processes, as well as the above patent, take advantage of the fact that all aromatic hydrocarbons are polar. That is to say, if a solvent capable of dissolving polar material, such as sulfolane, therein is added to a hydrocarbon mixture, polar aromatic hydrocarbons are selectively dissolved and thus separated from nonpolar non-aromatic hydrocarbons. This process has an advantage in that it is possible to produce a highly pure aromatic hydrocarbon mixture, but is disadvantageous in that an additional solvent extraction device is necessary and a solvent must be continuously supplied during operation. Accordingly, there remains a need for a process for separating aromatic hydrocarbons and non-aromatic hydrocarbons from feedstock oil without an additional solvent extraction step.

In connection with this, effort has been made to employ another reaction system instead of a solvent extraction process in order to separate aromatic compounds from non-aromatic compounds. The non-aromatic compounds which are mixed with the aromatic compounds are converted into gaseous hydrocarbons through a hydrocracking reaction using a catalyst, and the aromatic compounds and the non-aromatic compounds are separated from each other using a gas-liquid separator at a rear part of a reactor. This technology has been developed from U.S. Patent No. 3,729,409.

Furthermore, a process for producing aromatic hydrocarbons and LPG from a hydrocarbon mixture, in which aromatic compounds of the hydrocarbon mixture are converted into a fraction including benzene, toluene, xylene and the like through dealkylation and/or transalkylation reactions, and non-aromatic compounds are converted into gaseous material that is rich in LPG through a hydrocracking reaction, has been studied.

U.S. Patent No. 2002/0092797 discloses a process for converting aromatic compounds in hydrocarbon feedstock to oil components including BTX through dealkylation and/or transalkylation and for converting non-aromatic compounds to LPG-rich gaseous components through hydrocracking; in the presence of a catalyst. The hydrocarbon feedstock is reformate, pyrolysis gasoline, C9 or higher aromatic components-containing mixture, naphtha and mixture thereof. The catalyst comprises platinum/tin or platinum/lead and a mixture support consisting of 10-95 wt% of zeolite having a molar ratio of silica/alumina of 200 or less and 5-90 wt% of inorganic binder, said zeolite being selected from the group consisting of mordenite, beta type zeolite, ZSM-5 type zeolite.

The above-mentioned processes, respectively, which have the common object of producing aromatic hydrocarbon products, such as benzene, toluene, or xylene, have been independently developed as competitive, or complementary/substitution technologies. However, a process for improving productivity of aromatic hydrocarbons, particularly, benzene, by integrating competing processes has not yet been suggested.

### Disclosure of Invention

### Technical Problem

Leading to the present invention, the intensive and thorough research on production of benzene, carried out by the present inventors aiming to avoid the problems encountered in the prior arts, resulting in the finding that, when a process for producing an aromatic hydrocarbon mixture and LPG from a hydrocarbon mixture and a solvent extraction process for separating and recovering polar hydrocarbons from a hydrocarbon mixture are integrated, it is possible to improve the productivity and efficiency of each process or of the integrated process, thereby accomplishing the present invention.

Therefore, it is an object of the present invention to provide a process for increasing the productivity of products by integrating two processes which have different functions and compete with or complement each other.

It is another object of the present invention to provide a process for increasing the production of benzene from a hydrocarbon mixture so as to improve productivity.

### Technical Solution

In order to accomplish the above objects, there is provided a process, as described in claim 1, for increasing the production of benzene from a hydrocarbon mixture, comprising the following steps of:
(a) separating a hydrocarbon feedstock into a C6 or lower hydrocarbon stream and a C7 or higher hydrocarbon stream;
(b) separating the C6 or lower hydrocarbons into a non-aromatic hydrocarbon stream and an aromatic hydrocarbon stream through a solvent extraction process;
(c) recovering benzene from the aromatic hydrocarbon stream;
(d) feeding the C7 or higher hydrocarbons and hydrogen into at least one reaction area;
(e) converting the C7 or higher hydrocarbons in presence of a catalyst in the reaction area into (i) aromatic hydrocarbons which are rich in benzene, toluene, and xylene through dealkylation/transalkylation reactions, and (ii) non-aromatic hydrocarbons which are rich in liquefied petroleum gas through a hydrocracking reaction;
(f) separating reaction products of step (e) into an overhead stream, which contains hydrogen, methane, ethane, and the liquefied petroleum gas, and a bottom stream, which contains the aromatic hydrocarbons, and a small amount of hydrogen and non-aromatic hydrocarbons, using a gas-liquid separation process; and
(g) recovering benzene, toluene, xylene, and C9 or higher aromatic compounds, respectively from the bottom stream.

It is preferable that steps (c) and (g) be simultaneously conducted using a same device or be independently conducted using separately provided devices.

The process may further comprise recovering the liquefied petroleum gas from the overhead stream.

Preferably, 10-95 wt% zeolite, which is at least one selected from a group consisting of mordenite, a beta type of zeolite, and a ZSM-5 type of zeolite, and which has a silica/alumina molar ratio of 200 or less, is mixed with 5-90 wt% inorganic binder to produce a support, and platinum/tin or platinum/lead is supported on the mixture support to produce the catalyst of step (e).

Meanwhile, it is preferable that the hydrocarbon feedstock be selected from a group consisting of reformate, pyrolysis gasoline, desulfurized/denitrified fluidized catalytic cracking gasoline, C9+ aromatic-containing mixture, naphtha, and a mixture thereof.

### Advantageous Effects

In the present invention, after a hydrocarbon mixture is separated into a fraction in which the number of carbon atoms is 6 or lower and a residual fraction, hydrocarbons in which the number of carbon atoms is 7 or higher are used as a feedstock of a process for producing an aromatic hydrocarbon mixture and LPG, and hydrocarbons in which the number of carbon atoms is 6 or lower are fed as a feedstock of a solvent extraction process. Thereby, the mixture converted through the catalytic reaction, and the fraction which is separated through the extraction and is rich in benzene, toluene, and xylene, are separated into benzene, toluene, xylene, and C9+ aromatic compounds using a difference in boiling point and a separation device which includes a distillation column, resulting in the improved production of benzene.

Therefore, when the process for producing the highly pure aromatic hydrocarbon mixture, the LPG, and the non-aromatic hydrocarbons from the hydrocarbon feedstock, and the solvent extraction process for separating and recovering polar hydrocarbons from the hydrocarbon feedstock containing the polar hydrocarbons and nonpolar hydrocarbons are integrated according to the process of the present invention, it is possible to significantly improve the productivity of products in comparison with the separate use of each process.

### Brief Description of the Drawings

The above and other objects, features and advantages of the present invention will be more clearly understood from the following detailed description taken in conjunction with the accompanying drawings, in which:
FIG. 1 illustrates one embodiment of a procedure of increasing the production of benzene from a hydrocarbon mixture, according to the present invention; and
FIG. 2 illustrates another embodiment of a procedure of increasing the production of benzene from a hydrocarbon mixture, according to the present invention.

### Best Mode for Carrying Out the Invention

Hereinafter, a detailed description will be given of the present invention, referring to the drawings.

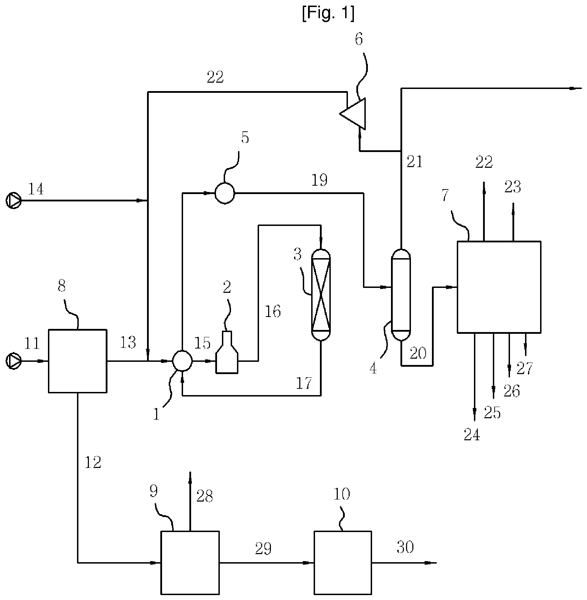

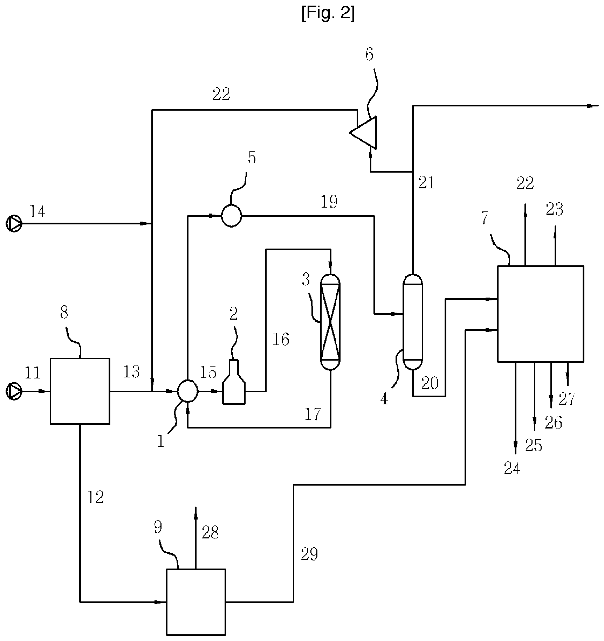

FIGS. 1 and 2 illustrate procedures of increasing the production of benzene from a hydrocarbon mixture, according to preferred embodiments of the present invention.

With reference to FIGS. 1 and 2, a hydrocarbon feedstock 11 as feedstock oil of a process according to the present invention is separated into a fraction 12 in which the number of carbon atoms is 6 or lower and a fraction 13 in which the number of carbon atoms is 7 or higher in a fractionation unit 8. The fraction 12 in which the number of carbon atoms is 6 or lower is fed as a feedstock for a solvent extraction process 9, and the fraction 13 in which the number of carbon atoms is 7 or higher is fed as a feedstock for a process of producing aromatic hydrocarbons and LPG from a hydrocarbon mixture.

The hydrocarbon feedstock used in the present invention preferably includes hydrocarbons having a boiling point of 30-250°C, and may be selected from the group consisting of reformate, pyrolysis gasoline, desulfurized/denitrified fluidized catalytic cracking gasoline, C9+ aromatic-containing mixture, naphtha, and a mixture thereof.

The fraction 13, in which the number of carbon atoms is 7 or higher and which is fed as the feedstock for the process of producing the aromatic hydrocarbons and the LPG from the hydrocarbon mixture, is mixed with circulating hydrogen 22 and highly pure hydrogen 14, and is then fed in a hydrogen/feedstock mixture form 15 into a reactor 3.

In connection with this, a separate heater 2 is provided in order to increase the temperature of the hydrogen/feedstock mixture to a reaction temperature. The hydrogen/feedstock mixture is heated to some extent 15 through heat exchange with reaction products 17 which are discharged from the reactor 3 and then fed into a heat exchanger 1, and is then fed into the heater 2.

The hydrogen/feedstock mixture 16 which is fed into the reactor 3 is subjected to dealkylation, transalkylation, and hydrogenation reactions in the presence of a catalyst.

That is to say, a hydrocracking reaction of non-aromatic hydrocarbon compounds, and the dealkylation and transalkylation reactions of aromatic hydrocarbon compounds are simultaneously carried out in the reactor 3 to produce main basic petrochemical materials, such as benzene, toluene, and xylene, and byproducts, such as LPG and non-aromatic compounds.

In connection with this, a catalyst, which is packed in the reactor 3 to cause the dealkylation, transalkylation, and hydrogenation reactions, is not limited as long as it is known to those skilled in the art, and, preferably, may be a catalyst disclosed in U.S. Patent No. 6,635,792.

That is to say, 10-95 wt% zeolite, which is at least one selected from the group consisting of mordenite, a beta type of zeolite, and a ZSM-5 type of zeolite and which has a silica/alumina molar ratio of 200 or less, is mixed with 5-90 wt% inorganic binder to produce a support, and platinum/tin or platinum/lead is supported on the mixture support, thereby the catalyst is created.

Meanwhile, the products 17 are present in a gaseous form at a relatively high temperature after the reactions are finished, are circulated into the heat exchanger 1 before they are fed into a gas-liquid separator 4, emit heat to the hydrogen/feedstock mixture therein, and are fed into a cooler 5.

A product stream 19 passing through the cooler 5 is fed into the gas-liquid separator 4 at about 30-50°C, and is then separated into a gaseous component and a liquid component.

The gaseous component is discharged in an overhead stream 21 from the gas-liquid separator 4, and the liquid component is discharged in a bottom stream 20 therefrom. In connection with this, the gaseous component 21 includes about 60-75 mol% hydrogen and 25-40 mol% hydrocarbon components, and the hydrocarbon components include methane, ethane, and LPG which have relatively small numbers of carbon atoms. The hydrogen component is compressed by a compressor 6, mixed with highly pure hydrogen 14 which is fed to control the purity of hydrogen, and is fed in conjunction with the feedstock 13 into a reaction area. Methane, ethane, and the LPG which are contained in the overhead stream 21 may selectively be recovered using an additional distillation process.

Meanwhile, the bottom stream 20 consists mostly of aromatic components, and also includes residual hydrogen and light non-aromatic components in a small amount. Accordingly, the liquid component is additionally subjected to a separation and purification process, and is separated into residual hydrogen 22, a non-aromatic component 23, and benzene 24, toluene 25, xylene 26, and C9+ aromatic compounds 27, which have purity of 99 % or more, using a difference in boiling point in a fractionation unit 7.

In summary, the hydrocarbon mixture, in which the number of carbon atoms is 7 or higher, is subjected to dealkylation, transalkylation, and hydrogenation reactions in the presence of the catalyst, thereby C9, C10, and C11 aromatic compounds are converted into benzene, toluene, and xylene.

Meanwhile, the fraction 12, which is separated by the fractionation unit 8 and is then fed as a feedstock of a solvent extraction process 9 and in which the number of carbon atoms is 6 or lower, is separated into non-aromatic hydrocarbons 28 which are nonpolar hydrocarbons and aromatic hydrocarbons 29 which are polar hydrocarbons.

As shown in FIG. 1, the aromatic hydrocarbons 29, which are the polar hydrocarbons, are fed into a fractionation unit 10 at a rear stage to produce benzene 30, or, as shown in FIG. 2, they are fed into the fractionation unit 7 of the process using the C7 or higher hydrocarbon mixture as a feedstock to produce benzene 24, toluene 25, and xylene 26 using a difference in boiling point.

As described above, separate processes which are integrated in the present invention have the common object of producing aromatic hydrocarbon products, such as benzene, toluene, and xylene. However, they are different from each other in that, in one process, the contents of benzene, toluene, and xylene in feedstock oil are changed through dealkylation and transalkylation reactions using the catalyst, while, in the other process, the contents of benzene, toluene, and xylene in feedstock oil are not changed.

In the present invention, the two separate processes are integrated, the hydrocarbon mixture is separated into the fraction in which the number of carbon atoms is 6 or lower and the fraction in which the number of carbon atoms is 7 or higher, and they are, respectively, used as a feedstock in the two processes. That is to say, the hydrocarbons in which the number of carbon atoms is 7 or higher are used as the feedstock of the process for producing the aromatic hydrocarbon mixture and the LPG, and the hydrocarbons in which the number of carbon atoms is 6 or lower are used as the feedstock of the solvent extraction process for separating and recovering the polar hydrocarbons from the hydrocarbons containing the polar hydrocarbons and the nonpolar hydrocarbons. Thereby, the mixture converted through the catalytic reaction, and the fraction which is separated through extraction and is rich in benzene, toluene, and xylene are separated into benzene, toluene, xylene, and C9+ aromatic compounds, respectively using a difference in boiling point through a separation device which includes a distillation column, resulting in the improved production of benzene.

Therefore, when the process for producing the highly pure aromatic hydrocarbon mixture, the LPG, and the non-aromatic hydrocarbons from the hydrocarbon feedstock, and the solvent extraction process for separating and recovering the polar hydrocarbons from the hydrocarbon feedstock containing the polar hydrocarbons and the nonpolar hydrocarbons are integrated according to the method of the present invention, it is possible to significantly improve the productivity of products in comparison with the separate use of each process.

### Mode for the Invention

A better understanding of the present invention may be obtained through the following examples and comparative examples which are set forth to illustrate, but are not to be construed as the limit of the present invention.

### EXAMPLE 1 AND COMPARATIVE EXAMPLES 1 AND 2

It is necessary to confirm the productivities of separate processes and the integrated process according to the process of the present invention, therefore tests were conducted to achieve the confirmation in the following examples.

### COMPARATIVE EXAMPLE 1

The productivity of products in a solvent extraction process using pyrolysis gasolines as a feedstock was confirmed, and the results are described in the following Table 1.

**Table 1**

| Feedstock | | Operation conditions | | | Result | | |
|---|---|---|---|---|---|---|---|
| Flow rate | Composition (wt%) | Temp. | Press. | Ratio | Product (kg/hr) | Extract composition (wt%) | Raffinate composition (wt%) |
| 10 kg/hr | C6 paraffin 4.48 | 90°C | 7kg/cm²g | 2 | Benzene 4.22 | C10+ paraffin 0.002 | C6 paraffin 25.421 |
| | C7 paraffin 2.58 | | | | Toluene 2.07 | C8 naphthene 0.001 | C7 paraffin 14.640 |
| | C8 paraffin 0.9 | | | | Xylene 0.67 | Benzene 51.466 | C8 paraffin 5.106 |
| | C9 paraffin 0.27 | | | | Total 6.96 | Toluene 25.260 | C9 paraffin 1.531 |
| | C10+ paraffin 1.85 | | | | | Ethyl benzene 8.198 | C10+ paraffin 10.487 |
| | C5 naphthene 2 | | | | | Xylene 8.684 | C5 naphthene 11.349 |
| | C6 naphthene 4.16 | | | | | C9+ aromatics 6.388 | C6 naphthene 23.605 |
| | C7 naphthene 0.61 | | | | | | C7 naphthene 3.461 |
| | C8 naphthene 0.47 | | | | | | C8 naphthene 2.664 |
| | Benzene 42.4 | | | | | | Benzene 0.024 |
| | Toluene 20.85 | | | | | | Toluene 0.237 |
| | Ethyl benzene 6.76 | | | | | | Ethyl benzene 0.038 |
| | Xylene 7.3 | | | | | | Xylene 0.828 |
| | C9+ aromatics 5.37 | | | | | | C9+ aromatics 0.609 |

Temp.: Extraction temperature
Press.: Extraction pressure
Ratio: Solvent/H.C. volume Ratio

### COMPARATIVE EXAMPLE 2

The productivity of a process for producing aromatic hydrocarbons and LPG from a hydrocarbon mixture using pyrolysis gasolines as a feedstock was confirmed, and the results are described in the following Table 2.

**Table 2**

| Feedstock | | Operation conditions | | | Result | |
|---|---|---|---|---|---|---|
| Flow rate | Composition (wt%) | Reaction Temp. | Reaction pressure | H₂/H.C. molar ratio | Product (kg/hr) | Composition (wt%) |
| 10 kg/hr | C6 paraffin 4.48 | 340°C | 30kg/cm²g | 4 | Benzene 1.93 | C1 paraffin 0.47 |
| | C7 paraffin 2.58 | | | | Toluene 3.71 | C2 paraffin 7.37 |
| | C8 paraffin 0.9 | | | | Xylene 2.18 | C3 paraffin 6.23 |
| | C9 paraffin 0.27 | | | | Total 7.82 | C4 paraffin 3.04 |
| | C10+ paraffin 1.85 | | | | | C5 paraffin 0.85 |
| | C5 naphthene 2 | | | | | C6 paraffin 0.11 |
| | C6 naphthene 4.16 | | | | | C7 paraffin 0.02 |
| | C7 naphthene 0.61 | | | | | C8 paraffin 0.02 |
| | C8 naphthene 0.47 | | | | | C9 paraffin 0.02 |
| | Benzene 42.4 | | | | | C6 naphthene 0.02 |
| | Toluene 20.85 | | | | | C7 naphthene 0.03 |
| | Ethyl benzene 6.76 | | | | | Benzene 19.31 |
| | Xylene 7.3 | | | | | Toluene 37.05 |
| | C9+ aromatics 5.37 | | | | | Xylene 21.84 |
| | | | | | | C9+ aromatic 5.64 |

### EXAMPLE 1

The productivity of the integrated process shown in FIG. 1 using pyrolysis gasolines as a feedstock was confirmed, and the results are described in the following Table 3.

**Table 3**

| | Solvent extraction | | Catalystic reaction | | Integration result | |
|---|---|---|---|---|---|---|
| Feedstock (wt%) | 4.94kg/hr | | 5.06kg/hr | | | |
| | C6 paraffin 9.07 | | C7 paraffin 0.12 | | | |
| | C7 paraffin 4.29 | | C8 paraffin 2.03 | | | |
| | Benzene 85.80 | | C9 paraffin 0.6 | | | |
| | Toluene 0.84 | | C10+ paraffin 4.15 | | | |
| | | | C6 naphthene 0.07 | | | |
| | | | C7 naphthene 0.68 | | | |
| | | | C8 naphthene 1.07 | | | |
| | | | Benzene 0.8 | | | |
| | | | Toluene 46.8 | | | |
| | | | Ethyl benzene 15.2 | | | |
| | | | Xylene 16.4 | | | |
| | | | C9+ aromatics 12.08 | | | |
| Operation conditions | Extraction temp. | 90°C | Reaction temp. | 340°C | | |
| | Extraction press. | 7kg/cm²g | Reaction press. | 30kg/cm²g | | |
| | Solvent/H.C. volume ratio | 2 | H₂/H.C. molar ratio | 4 | | |
| Result | Benzene | 4.15kg/hr | Benzene | 0.97kg/hr | Benzene | 5.12kg/hr |
| | Toluene | 0.04kg/hr | Toluene | 1.86kg/hr | Toluene | 1.90kg/hr |
| | Total | 4.19kg/hr | Xylene | 1.09kg/hr | Xylene | 1.09kg/hr |
| | | | Total | 3.92kg/hr | Total | 8.11kg/hr |

The present invention has been described in an illustrative manner, and it is to be understood that the terminology used is intended to be in the nature of description rather than of limitation. Many modifications and variations of the present invention are possible in light of the above teachings. Therefore, it is to be understood that within the scope of the appended claims, the invention may be practiced otherwise than as specifically described.

## Claims

1. A process for increasing production of benzene from a hydrocarbon mixture, comprising:
(a) separating the hydrocarbon feedstock into a C6 or lower hydrocarbon stream and a C7 or higher hydrocarbon stream;
(b) separating the C6 or lower hydrocarbons, coming from step (a) into a non-aromatic hydrocarbon stream and an aromatic hydrocarbon stream through a solvent extraction process;
(c) recovering benzene from the aromatic hydrocarbon stream;
(d) feeding the C7 or higher hydrocarbons and hydrogen into at least one reaction area;
(e) converting the C7 or higher hydrocarbons coming from step (a) in presence of a catalyst in the reaction area into (i) aromatic hydrocarbons, which are rich in benzene, toluene, and xylene employing dealkylation/transalkylation reactions, and (ii) non-aromatic hydrocarbons which are rich in liquefied petroleum gas through a hydrocracking reaction;
(f) separating reaction products of step (e) into an overhead stream, which contains hydrogen, methane, ethane, and the liquefied petroleum gas, and a bottom stream, which contains the aromatic hydrocarbons, and a small amount of hydrogen and non-aromatic hydrocarbons, using a gas-liquid separation process; and
(g) recovering benzene, toluene, xylene, and C9 or higher aromatic compounds from the bottom stream.

2. The process as set forth in claim 1, wherein steps (c) and (g) are simultaneously conducted using a same device or are independently conducted using separately provided devices.

3. The process as set forth in claim 1, further comprising recovering the liquefied petroleum gas from the overhead stream.

4. The process as set forth in claim 1, wherein 10 - 95 wt% zeolite, which is at least one selected from a group consisting of mordenite, a beta type of zeolite, and a ZSM-5 type of zeolite, and which has a silica/alumina molar ratio of 200 or less, is mixed with 5 - 90 wt% inorganic binder to produce a support, and platinum/tin or platinum/lead is supported on the mixture support to produce the catalyst of step (e).

5. The process as set forth in claim 1, wherein the hydrocarbon feedstock is selected from a group consisting of reformate, pyrolysis gasoline , desulfurized/denitrified fluidized catalytic cracking gasoline, a C9+ aromatic-containing mixture, naphtha, and a mixture thereof.

## Patentansprüche

1. Verfahren zur Erhöhung der Produktion von Benzol aus einem Kohlenwasserstoffgemisch, umfassend:
(a) Trennen des Kohlenwasserstoffausgangsmaterials in einen C6- oder geringeren Kohlenwasserstoffstrom und einen C7- oder höheren Kohlenwasserstoffstrom;
(b) Trennen der aus dem Schritt (a) gewonnenen C6- oder geringeren Kohlenwasserstoffe in einen nichtaromatischen Kohlenwasserstoffstrom und einen aromatischen Kohlenwasserstoffstrom durch ein Lösungsmittelextraktionsverfahren;
(c) Rückgewinnen von Benzol aus dem aromatischen Kohlenwasserstoffstrom;
(d) Zuführen der C7- oder höheren Kohlenwasserstoffe und des Wasserstoffs in wenigstens einen Reaktionsbereich;
(e) Umwandeln der aus Schritt (a) gewonnenen C7- oder höheren Kohlenwasserstoffe in Gegenwart eines Katalysators im Reaktionsbereich in (i) aromatische Kohlenwasserstoffe, die reich an Benzol, Toluol und Xylol sind, durch Dealkylierungs-/Transalkylierungsreaktionen und in (ii) nichtaromatische Kohlenwasserstoffe, die reich an verflüssigtem Erdölgas sind, durch eine Hydrocrackreaktion;
(f) Trennen der Reaktionsprodukte aus Schritt (e) in einen Kopfstrom, der Wasserstoff, Methan, Ethan und verflüssigtes Erdölgas enthält, und einen Bodenstrom, der die aromatischen Kohlenwasserstoffe und eine geringe Menge an Wasserstoff und nichtaromatischen Kohlenwasserstoffen enthält, durch Einsatz eines Gas-Flüssigkeitstrennungsverfahrens; und
(g) Rückgewinnen von Benzol, Toluol, Xylol und C9- oder höheren aromatischen Verbindungen aus dem Bodenstrom.

2. Verfahren nach Anspruch 1, wobei die Schritte (c) und (g) gleichzeitig durch Verwenden einer gleichen Einrichtung oder unabhängig voneinander durch Verwenden von getrennt bereitgestellten Einrichtungen durchgeführt werden.

3. Verfahren nach Anspruch 1, ferner umfassend das Rückgewinnen des verflüssigten Erdölgases aus dem Kopfstrom.

4. Verfahren nach Anspruch 1, wobei 10-95 Gew.-% Zeolith, der wenigstens aus einer Gruppe bestehend aus Mordenith, einem Zeolith vom Beta-Typ und einem Zeolith vom ZSM-5-Typ ausgewählt ist und ein Molverhältnis von Siliciumdioxid zu Aluminiumoxid von 200 oder weniger aufweist, mit 5-90 Gew.-% anorganischen Bindemittels zur Herstellung eines Trägers gemischt wird, und Platin/Zinn oder Platin/Blei auf dem Gemischträger getragen wird, um den Katalysator des Schritts (e) zu erzeugen.

5. Verfahren nach Anspruch 1, wobei das Kohlenwasserstoffausgangsmaterial ausgewählt ist aus einer Gruppe bestehend aus Reformat, Pyrolysebenzin, entschwefeltem/denitrifiziertem, fluidisiertem, katalytischem Crackbenzin, einem C9+ aromatenhaltigen Gemisch, Rohbenzin und Gemischen davon.

## Revendications

1. Procédé pour augmenter la production de benzène à partir d'un mélange d'hydrocarbures, comprenant :
(a) la séparation de la charge d'alimentation d'hydrocarbures en un courant d'hydrocarbures en C6 ou inférieurs et un courant d'hydrocarbures en C7 ou supérieurs ;
(b) la séparation des hydrocarbures en C6 ou inférieurs, provenant de l'étape (a) en un courant d'hydrocarbures non aromatiques et un courant d'hydrocarbures aromatiques par le biais d'un processus d'extraction par solvant ;
(c) la récupération de benzène à partir du courant d'hydrocarbures aromatiques ;
(d) l'alimentation des hydrocarbures en C7 ou supérieurs et d'hydrogène dans au moins une zone de réaction ;
(e) la conversion des hydrocarbures en C7 ou supérieurs provenant de l'étape (a) en présence d'un catalyseur dans la zone de réaction en (i) hydrocarbures aromatiques qui sont riches en benzène, toluène et xylène par le biais de réactions de désalkylation/transalkylation, et en (ii) hydrocarbures non aromatiques qui sont riches en gaz de pétrole liquéfié par le biais d'une réaction d'hydrocracking ;
(f) la séparation des produits de réaction de l'étape (e) en un courant de tête, qui contient de l'hydrogène, du méthane, de l'éthane et le gaz de pétrole liquéfié, et un courant de queue, qui contient les hydrocarbures aromatiques, et une petite quantité d'hydrogène, et les hydrocarbures non aromatiques, en utilisant un processus de séparation gaz-liquide ; et
(g) la récupération de benzène, de toluène, de xylène et de composés aromatiques en C9 ou supérieurs, à partir du courant de queue.

2. Procédé selon la revendication 1, dans lequel les étapes (c) et (g) sont exécutées simultanément en utilisant un même dispositif ou sont exécutées indépendamment en utilisant des dispositifs prévus séparément.

3. Procédé selon la revendication 1, comprenant en outre la récupération du gaz de pétrole liquéfié à partir du courant de tête.

4. Procédé selon la revendication 1, dans lequel 10-95% en poids de zéolite, qui est au moins une sélectionnée à partir d'un groupe comprenant la mordénite, un type bêta de zéolite et un type ZSM-5 de zéolite, et qui a un rapport molaire de silice/alumine de 200 ou moins, sont mélangés avec 5-90% en poids de liant inorganique pour produire un support, et du platine/étain ou platine/plomb est supporté sur le support de mélange pour produire le catalyseur de l'étape (e).

5. Procédé selon la revendication 1, dans lequel la charge d'alimentation d'hydrocarbures est sélectionnée à partir d'un groupe comprenant du reformat, de l'essence de pyrolyse, de l'essence de craquage catalytique fluidisé désulfurée/dénitrifiée, un mélange contenant des aromatiques en C9+, du naphta, et un mélange de ceux-ci.
